Europäisches Patentamt

European Patent Office

Office européen des brevets

(11) Publication number: **0 419 753 A1**

# EUROPEAN PATENT APPLICATION

(21) Application number: 89830424.1

(22) Date of filing: 28.09.89

(51) Int. Cl.5: **H05G 1/46, G01N 33/44**

(43) Date of publication of application:
03.04.91 Bulletin 91/14

(84) Designated Contracting States:
AT BE CH DE ES FR GB GR IT LI LU NL SE

(71) Applicant: GOVONI SPA
Via Bondenese, 12
Casumaro Ferrara(IT)

(72) Inventor: Gulmini, Carlo
via Ex-dogana 7
Casumaro (Ferrara)(IT)

(74) Representative: Sassatelli, Franco T., Dr.
c/o INIP via Ruggi 5
I-40137 Bologna(IT)

(54) Recognition system for the chemical constitution of plastic materials by means of electromagnetic waves.

(57) The identification system essentially consists of an emission source (1) of electromagnetic waves which are sent towards a receiver-sensor (2) fitted at a distance determined in advance. In absence of an interposed body the energy level reaching the receiver-sensor (2) is the reference measurement. Owing to the interposition between the emission source (1) and the receiver-sensor (2) of a plastic material body (3), such as polypropylene, polythene, polystyrene, vinyl chloride resin and others, a specific and charateristic modification is obtained of the energy level that can be observed for each type ofr different interposed plastic material, by reading the voltage level supplied by the receiver-sensor (2).

FIG.1

EP 0 419 753 A1

## RECOGNITION SYSTEM FOR THE CHEMICAL CONSTITUTION OF PLASTIC MATERIALS BY MEANS OF ELECTROMAGNETIC WAVES.

The invention refers to the recognizing method of the chemical constitution of plastic materials which uses the electromagnetic waves supplied by an emission source. The procedure starts from the presupposition that by interposing a body in plastic material between the emission source and the receiver-sensor, is going through the part, the quantity of energy is modified which is supplied by the waves that reach the receiver-sensor. Therefore, to proceede to recognize the chemical constitution of any plastic material, its characteristic induced modification should be observed. The tracking of the absorption of the plastic material of the body thus interposed will allow, by means of an elaboration, if required through a statistic model, to work out indicative parameters for the specific type of the product. The pointed out parameter is transformed into an electrical impulse and then used to identify the nature and destination of the analysed object.

The identification system essentially consists of an emission source 1 of electromagnetic waves which are sent to a receiver-sensor 2 fitted at a distance fixed in advance. In absence of an interposed body, the energy level reaching the receiver-sensor 2 is the measurement reference. Owing to the interposition , between the emission source 1 and the receiver-sensor 2, of a body in plastic material 3, such as polypropylene, polythene, polystyrene, vinyl chloride resin and others, a specific and characteristic modification is obtained in the energy level that can be noted for each type of different interposed plastic material, by reading the voltage level supplied by the receiver-sensor 2 on the indicator 4.

With a view to better characterizing the typology of the examined plastic material, the quantity and quality of the used electromagnetic waves can be modified. These ones are characterized by a quantity bound to the m.A. and by an intensity bound to the K.V. Therefore, by changing one of the two above values (m.A. and K.V.) and by maintaining the other constant, it is possibile to obtain curves that are characteristic of each type of plastic material which has been put in.

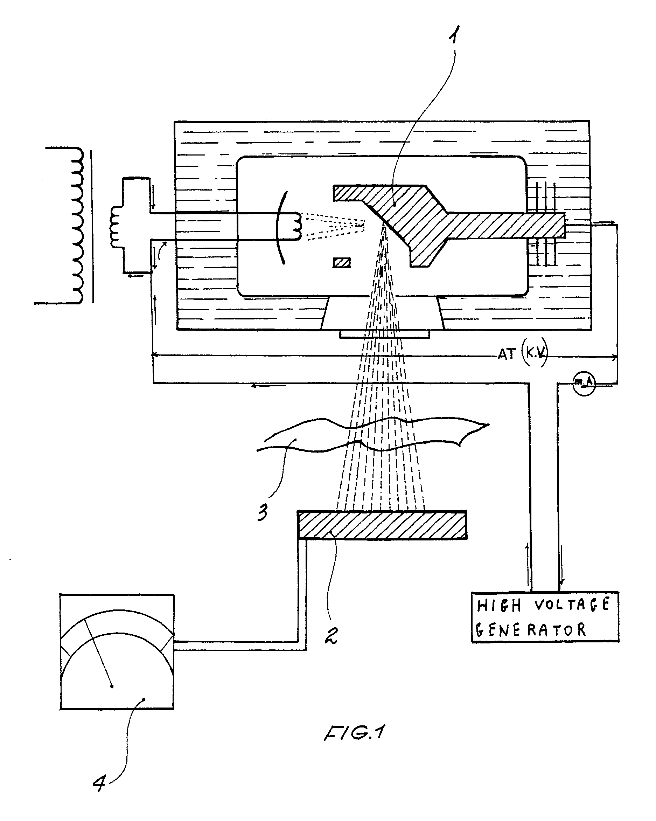

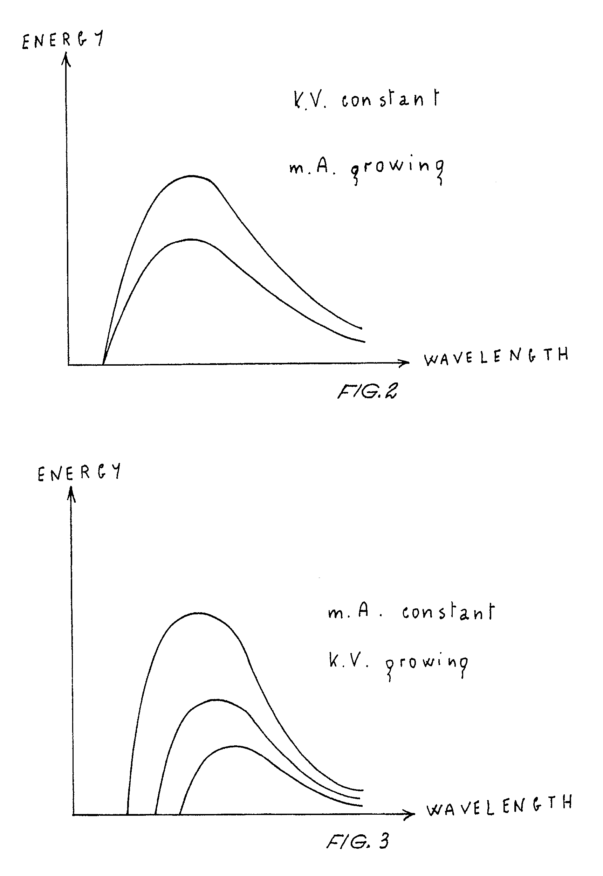

Forms performed according to the proceeding are illustrated, in a merely indicative way, in the drawings of tables 1 and 2. With reference to table 1, fig. 1 is the scheme of the recognizing system with interposed body which can be noted. With reference of table 2, fig. 2 is the scheme showing the state of the radius energy and of the wave length according to the changing m.A. and K.V. constant. Fig. 3 is the scheme representing the energy level and of the wave length according to the changing K.V. supplied to the emitter of constant m.A.

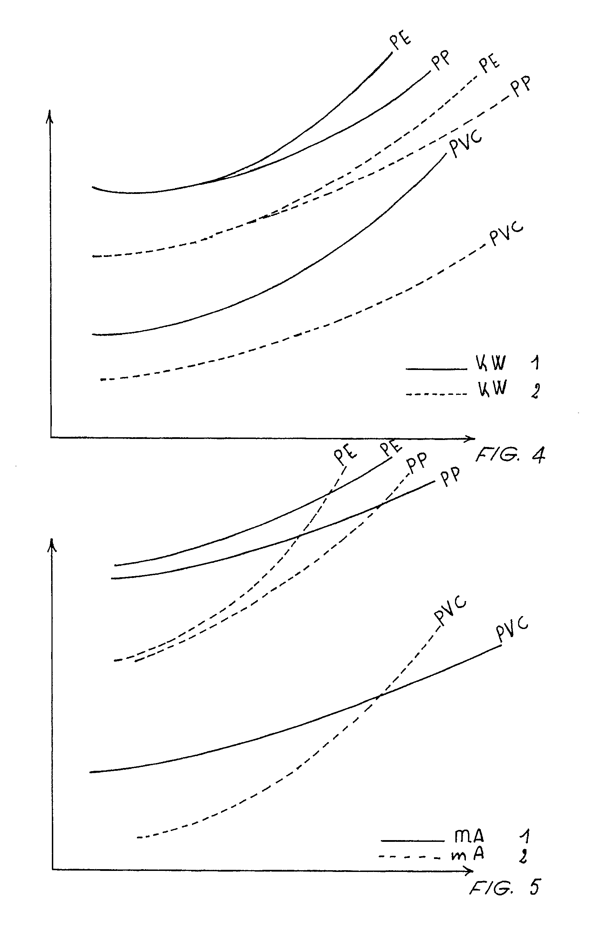

Within the limits of the employ of the above recognizing system, it may be required in advance to know which is the couple or couples of value (m.A. and K.V.) that enable at the highest to make evident the differences of the chemical composition of the plastic material object of the search. This is performed by drawing some curves on the two cartesian axis which respectively bring the m.A. into X-coordinate, as illustrated in table 3 - fig. 4, and the K.V., as illustrated in fig. 5 of the same table, and the percentage energy received by probe 2. In particular, in the first of the two diagrams, with the increase of the m.A. to constant K.V., a differentiation of the energy level is obtained which, when picked, points out the nature of the material. In an analogous way, fig. 5 shows that the energy reaching the sensor, according to the different used K.V., is different as to the different molecular composition of the analized plastic material.

The methodology can be universally used in both fixed and mobile type stations, for carrying out devices and plants of different kinds and to be used in any possibile industrial process.

## Claims

1) Recognition system for the chemical constitution of plastic materials by means of electromagnetic waves, characterized by the fact that the identification system essentially consists of an emission source (1) of electromagentic waves which are sent towards a receiver-sensor (2) fitted at a distance fixed in advance. In absence of an interposed body, the energy level which reaches the receiver-sensor (2) is the reference measurement. Owing to the interposition between the emission current (1) and the receiver-sensor (2) of a plastic material body (3), a specific modification is obtained which is characteristic of the energy level that can be observed for each type of interposed plastic material by reading the voltage level given by the receiver-sensor on the display (4). In order to characterize the typology of the examined plastic material, it is possibile to let the quantity and quality change of the electromagnetic waves which have been used. These ones are characterized by a quantity bound to the m.A. and by and intensity bound to the K.V.. In this connection, by letting one of the two sizes (m.A. and K.V.) change and by maintaining constant the other one, it is allowed to obtain some curves which are characteristic for each type of the plastic material that has been put in.

2) Recognition system for the chemical constitution of plastic materials by means of electromagnetic waves, as per claim 1), characterized by the fact that within the limits of the above recognizing system use, it could be required in advance to know which is the couple or couples of values (m.A. and K.V.) which allow to make evident at the highest degree the differences of the plastic material chemical composition, object of the investigation. This can be done by marking some curves on two cartesian axis which give in X-coordinate rispectively the m.A., the K.V. and the percentage energy received from the probe (2). In particular, in the first of the above mentioned diagram, when the m.A. are increasing with K.V. constant, a differentiation of the energy levels occurs which, after having been noted, states the nature of the product. Likewise, when the K.V. change, the energy which reaches the sensor is different according to the different molecular composition of the analysed plastic material.

1

AT (K.V.)

m.A

3

2

HIGH VOLTAGE
GENERATOR

4

FIG.1

4

ENERGY

K.V. constant

m.A. growing

WAVELENGTH

FIG.2

ENERGY

m.A. constant

K.V. growing

WAVELENGTH

FIG. 3

FIG. 4

FIG. 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 126 434 (KABUSHIKI KAISHA TOSHIBA) * abstract; page 1, line 1 - page 2, line 11; page 3, line 22 - page 4, line 32; page 11, lines 7-16; figure 1 * | 1,2 | H 05 G 1/46 G 01 N 33/44 |
| Y | PATENT ABSTRACTS OF JAPAN vol. 7, no. 253 (P-235)(1398), 10 November 1983; & JP - A - 58 137 771 (FURUKAWA DENKI KOGYO K.K.) 16.08.1983 | 1,2 | |
| Y | FR-A-2 528 176 (H. RITZL) * page 1, line 1 - page 2, line 4 * | 1,2 | |
| A | PATENT ABSTRACTS OF JAPAN vol. 12, no. 436 (P-787)(3283), 17 November 1988; & JP - A - 63 165 742 (SHIMADZU CORPORATION) 09.07.1988 | 1,2 | |
| A | GB-A-1 453 074 (ELIN UNION AG FUER ELEKTRISCHE INDUSTRIE) * page 1, line 70 - page 4, line 63; figures 1-5 * | 1,2 | TECHNICAL FIELDS SEARCHED (Int. Cl.5) |
| A | US-A-4 638 500 (W.F. SMITS et al.) * abstract; claims 1-6; figures 1,5 * | 1,2 | G 01 N 21/00 H 05 G 1/00 G 01 N 23/00 |

The present search report has been drawn up for all claims

| Place of search | Date of completion of the search | Examiner |
|---|---|---|
| BERLIN | 10-05-1990 | MOUTARD P.J. |

EPO FORM 1503 03.82 (P0401)